# EUROPEAN PATENT SPECIFICATION

(11) **EP 4 294 294 B1**
(45) Date of publication and mention of the grant of the patent: **16.07.2025**
(21) Application number: 22704571.3
(22) Date of filing: 07.02.2022
(51) Int. Cl.: A61B 17/29, A61B 34/00

(54) **JAW ACTUATION MECHANISM**
BACKENBETÄTIGUNGSMECHANISMUS
MÉCANISME D'ACTIONNEMENT DE LA MÂCHOIRE

(30) Priority: 19.02.2021 GB 202102359
(43) Date of publication of application: 27.12.2023
(73) Proprietor: Precision Robotics Limited, London W12 0BZ (GB)
(72) Inventor: SHANG, Jianzhong, Dartford, Kent DA1 3HZ (GB)
(74) Representative: Nederlandsch Octrooibureau
(86) International application number: PCT/GB2022/050317
(87) International publication number: WO 2022/175642

(56) References cited:
- WO-A1-2015/088647
- WO-A2-2016/025134
- US-A1- 2021 045 765

## Description

This invention relates to an actuation mechanism, and specifically, but not exclusively to an actuation mechanism which facilitates the actuation of two jaw members with a single pair of tendons. The invention has particular application in the field of surgical robotics to facilitate the actuation of two jaw members forming part of a surgical instrument end effector for minimally invasive surgical procedures, although the invention is not limited to such application and may also have use in other medical/surgical devices or other robotic devices comprising a tendon driven end effector with two or more movable parts, such as jaw members.

From herein the invention is primarily described in relation to application in the field of surgical robotics. However, this is for demonstrative purposes only and is not to the exclusion of the invention's application in other fields.

Known surgical instruments forming part of robotic surgical systems comprise a shaft, an articulation portion and an end effector. The shaft may extend from other components of the surgical robot which control and drive movement of the articulation portion and the end effector. The shaft may thereby facilitate positioning of the articulation portion and end effector at the required area of a patient. The articulation portion may comprise a plurality of joints positioned adjacent to one another to provide degrees of freedom of movement to the end effector relative to the shaft. Lastly, the end effector may be adapted to carry out particular actions required in surgical procedures.

Known end effectors include end effectors with two movable jaw members such as graspers, forceps, scissors and dissectors, for example. Known end effectors are also tendon driven meaning that each possible articulation of the end effector is caused by the pulling of a tendon which is anchored to the, or part of the, end effector. Further, known end effectors are driven by antagonistic pairs of tendons to ensure that each articulation may be reversed. For example, if the actuation of a jaw member in a first direction is caused by the pulling of a first tendon then actuation of the jaw member in a second, opposite direction may require the pulling of a second (antagonistic) tendon.

WO 2015/088647 A1 discloses an end effector of a surgical tool comprising a pair of jaws which are separately and independently pivotally connected to support plates and each of which privots about a different pivot point that defines a different pivot aixs, and curved cam slots extending along the circumference of a cam pull, which are connected to respective jaws via pins for actuating the jaws to rotate.

US 2021/045765 A1 discloses a jaw closure transmission system comprising a movable jaw configured to be actuated to rotate around a movable jaw pivot pint and a fixed jaw which is configured not to rotate, wherein a driving slot is only provided for the movable jaw.

WO 2016/025134 A2 discloses an end effector of a surgical tool comprising a pair of jaws which are separately and independently pivotally connected and each of which pivots about a different pivot point than the drive wheel which defines a different pivot axis, and arcuate cam slots on the respective jaws engaged with pins on the drive wheel for actuating the jaws to rotate.

In some known end effectors, each jaw member may be driven by a separate pair of antagonistic tendons. However, this means that four tendons are required for operation of the end effector and each tendon will have associated moving parts that drive actuation of the tendon and translate the movement of the tendons to the respective jaw member of the end effector. Such known end effectors may suffer limitations such as how small the surgical instrument can be to facilitate all four tendons and associated moving parts and how cost effectively it may be manufactured while comprising all of the necessary motors and other components to drive those four tendons.

To solve the technical problems above, the invention provides an actuation mechanism and a surgical instrument comprising the actuation mechanism according to the independent claims 1 and 10.

Further embodiments are specified in the dependent claims. No surgical methods are claimed.

According to a first aspect of the invention there is provided an actuation mechanism comprising a first jaw rotatable about a jaw axis and comprising a first jaw engagement portion, a second jaw rotatable about the jaw axis and comprising a second jaw engagement portion, and a driver rotatable about a drive axis parallel to the jaw axis and comprising a first drive engagement portion and a second drive engagement portion; wherein the first jaw engagement portion and the first drive engagement portion are slidably engageable with one another and the second jaw engagement portion and the second drive engagement portion are slidably engageable with one another; whereby, when the first jaw engagement portion is engaged with the first drive engagement portion and the second jaw engagement portion is engaged with the second drive engagement portion, rotation of the driver about the drive axis causes rotation of the first jaw in a first sense and the second jaw in an opposite sense, wherein one of the first jaw engagement portion and the first drive engagement portion comprises a first pin, and the other one comprises a first radially extending slot; and one of the second jaw engagement portion and the second drive engagement portion comprises a second pin, and the other one comprises a second radially extending slot.

Rotation of each jaw may be caused by rotation of the driver by virtue of the configuration of each jaw engagement portion relative to its respective drive engagement portion. For example, the first jaw engagement portion may be a fixed distance from the jaw axis and the first drive engagement portion may be arranged such that as it rotates about the drive axis it causes the first jaw engagement portion to rotate about the jaw axis while sliding against the first drive engagement portion. Meanwhile, the second jaw engagement portion and second drive engagement portion may be complimentarily configured such that the same rotation of the driver causes an equal and opposite rotation of the second jaw.

Hence, by means of the invention both the first jaw and the second jaw of the actuation mechanism may be rotated or actuated simultaneously by rotating the single driver. Rotation of the driver may be driven by any suitable means such as via a motor driven axis fixed to the driver or via a pair of antagonistic tendons each attached to the driver.

In embodiments of the invention, the actuation mechanism may further comprise a first and second tendon each attachable to the driver.

In such embodiments of the invention the first and second tendons may form a pair of antagonistic tendons wherein actuation of the first tendon causes rotation of the driver in a first sense, and actuation of the second tendon causes rotation of the driver disc in a opposite sense. The actuation mechanism may therefore be operated by actuating the single pair of antagonistic tendons. In other words, there is no requirement for two separate pairs of antagonistic tendons to operate the actuation mechanism (one for each jaw) as is the case for some known end effectors.

In some embodiments of the invention, the first and second tendons may be joined and may further be integrally formed.

In embodiments of the invention, each jaw may comprise a drive portion, a tool portion and a jaw axle receiving portion positioned between the drive portion and the tool portion and extending along the jaw axis.

In such embodiments of the invention the jaw axle receiving portion may comprise an aperture extending along the jaw axis that may facilitate rotation of the jaw about the jaw axis. The tool portion may extend from the jaw axle receiving portion in a first direction normal to the jaw axis and the drive portion may extend in a second direction substantially opposite to the first direction. In some embodiments, the tool portion may be integral with the drive portion and in other embodiments the tool portion may be separate but attachable to the drive portion.

In embodiments of the invention, the the first and second jaw engagement portions may be positioned in the drive portion of the respective jaw.

In such embodiments of the invention the drive portion of each jaw may be positionable to overlap, at least partially, the driver suitably for the respective jaw and drive engagement portions to engage with one another.

In embodiments of the invention, the tool portions of the first and second jaws may be configured as scissor blades, forceps, dissectors or graspers. Further, in embodiments of the invention, the tool portions may be configured to form any suitable bipolar tool which may benefit from being driven by a single driver and, optionally, a single pair of antagonistic tendons. Accordingly actuation mechanisms according to the invention may be adapted to provide a range of devices that are suitable for performing different tasks.

In embodiments of the invention, each jaw may be rotatable between an open position and a closed position. Further the actuation mechanism may be moveable between an open configuration, in which each jaw is in its open position, and a closed configuration, in which each jaw is in its closed position. In other words, the jaws may be open and closed by rotating the driver. The driver and the two jaws may be continuously rotatable such that each jaw may be rotated to any position between the respective open and closed positions.

In embodiments of the invention, the actuation mechanism may further comprise a housing comprising a jaw axle extending along the jaw axis and rotatably receivable within the jaw axle receiving portion of each jaw such that each jaw is rotatable relative to the housing about the jaw axle.

In such embodiments of the invention, the housing may be adapted to support each jaw and may, at least partially, conceal and protect the drive portion to reduce the risk of the pin and slot mechanisms becoming blocked or obstructed.

The housing may also be adapted to have a substantially smooth, regular and/or continual outer surface with minimal sharp edges or corners that may damage internal tissues of a patient if the actuation mechanism is in use as part of a surgical instrument or robot. The housing may also be configured to protect internal tissues of a patient from irregular surfaces of the driver, drive portions and engagement portions forming parts of the actuation mechanism.

In embodiments of the invention, the driver may further comprise a drive axle receiving portion and the housing may further comprise a drive axle extending along the drive axis and rotatably receivable within the drive axle receiving portion of the driver such that the driver is rotatable relative to the housing about the drive axle.

In such embodiments of the invention, the housing may be adapted to support the driver and may, at least partially, conceal and protect the driver and the attached first and second tendons to reduce the risk of the driver and/or tendons becoming blocked obstructed or damaged.

In embodiments of the invention, the first jaw engagement portion may comprise a first pin, the second jaw engagement portion may comprise a second pin, the first drive engagement portion may comprise a first slot and the second drive engagement portion may comprise a second slot.

In such embodiments of the invention, each slot may be a fixed distance from the drive axle receiving portion and hence at a fixed distance from the drive axis. In use, rotation of the driver about the drive axis causes the first and second slots to rotate about the drive axis. As each of the slots rotates, it causes the respective pin to move in order that it stays within the slot. Each pin may be a fixed distance from the jaw axle receiving portion (and hence a fixed distance from the jaw axis). This means that movement of the pin is restricted to rotation about the jaw axis while the pin is sliding within its respective slot. As each pin rotates about the jaw axis, each jaw is caused to rotate about the jaw axis accordingly. Hence rotation of the driver causes rotation of the jaws. Each pin may be positioned relative to the jaw axle receiving portion and each slot may be positioned and oriented relative to the drive axle receiving portion such that the rotation of each jaw caused by the driver is in an opposite direction to the direction of rotation of the other jaw. In some embodiments of the invention the degree of rotation of each jaw caused by the driver may be equal to the other jaw. In other words, the jaws may be movable substantially symmetrically to one another. In other embodiments of the invention the driver may cause the jaws to rotate different amounts, i.e. asymmetrically to one another.

In embodiments of the invention, the first jaw engagement portion may comprise a first slot, the second jaw engagement portion may comprise a second slot, the first drive engagement portion may comprise a first pin and the second drive engagement portion may comprise a second pin.

In such embodiments of the invention, each pin may be a fixed distance from the drive axle receiving portion, and hence a fixed distance from the drive axis. In use, rotation of the driver about the drive axis causes the first and second pins to rotate about the drive axis. As each of the pins rotates, it causes the respective slot to move in order that the pin stays within it. Each slot may be a fixed distance from the jaw axle receiving portion, and hence at a fixed distance from the jaw axis. This means that movement of the slot is restricted to rotation about the jaw axis while sliding to retain the respective pin within it. As each slot rotates about the jaw axis, each jaw is caused to rotate about the jaw axis accordingly. Hence rotation of the driver causes rotation of the jaws. Each pin may be positioned relative to the drive axle receiving portion and each slot may be positioned and oriented relative to the jaw axle receiving portion such that the rotation of each jaw caused by the driver is in an opposite direction to the direction of rotation of the other jaw. In some embodiments of the invention the degree of rotation of each jaw caused by the driver may be equal to the other jaw. In other words, the jaws may be movable substantially symmetrically to one another. In other embodiments of the invention the driver may cause the jaws to rotate different amounts, i.e. asymmetrically to one another.

In embodiments of the invention, the first jaw engagement portion may comprise a first pin, the first drive engagement portion may comprise a first slot, the second jaw engagement portion may comprise a second slot and the second drive engagement portion may comprise a second pin. Each pin and slot arrangement may operate as set out above.

The position of each pin and the position and orientation of each slot may be varied regardless of whether it is a jaw engagement portion or a drive engagement portion. Further, the performance of the actuation mechanism in use may be changed, tuned and/or optimised by adapting the position and/or orientation of the pins and slots. For example, the ratio between the radius of the driver and the distance between each pin and the drive axis is proportional to a force amplification ratio for the respective jaw. In other words, the closer that a pin is to the drive axis, the greater the force that may be applied to the pin by rotating the driver and the greater the force that the respective jaw may exert. Hence, the force exerted by a jaw when opening or closing from a given position may be increased or decreased by configuring the respective pin so that it is close to, or far way from, the drive axis when the respective jaw is in that position.

Also, the force that may be exerted by a slot on a pin is dependent on how close to parallel the slot is with a centre line of the respective jaw (i.e. a jaw centre line), wherein the jaw centreline extends through the jaw axis and the centre of the respective pin. If the slot is substantially parallel with the jaw centre line, forces exerted between the slot and the pin will be substantially normal to the jaw centre line and the proportion of force lost to friction due to the angles involved will be low. Conversely, if the slot is close to perpendicular with the jaw centre line, forces exerted between the slot and the pin will be at acute angles and will suffer losses in the amount of force transmitted to the jaw as a result.

According to a second aspect of the invention there is provided a surgical instrument comprising a shaft, an articulated portion coupled to the shaft and an end effector coupled to the articulated portion, which end effector comprises an actuation mechanism according to the first aspect of the invention.

The surgical instrument may be used for surgical procedures and may be a robotic surgical instrument. The articulated portion may be actuated to facilitate movement of the end effector relative to the shaft with up to six degrees of freedom. Further, the articulated portion may be actuated by a plurality of tendons which extend along the shaft to actuators and motors, for example, which may drive actuation of the tendons.

In use the end effector, articulated portion and shaft may be positioned as required relative to the patient such that the end effector may be manipulated (via actuation of the articulated portion) and actuated via the actuation mechanism, more particularly by rotation of the driver, to perform tasks required for the relevant surgical procedure.

The invention will now be described by way of example only with reference to the accompanying drawings in which:
Figure 1 is a schematic representation of an actuation mechanism according to an embodiment of the first aspect of the invention;
Figure 2 is a schematic representation of the actuation mechanism shown in Figure 1 with jaws in open positions;
Figure 3 is a rear view of the actuation mechanism shown in Figure 2;
Figure 4 is a schematic representation of the actuation mechanism shown in Figure 1 with jaws in closed positions;
Figure 5 is a rear view of the actuation mechanism shown in Figure 4;
Figures 6a, 6b and 6c are schematic representations of an actuation mechanism according to another embodiment of the first aspect of the invention showing front, back and side views respectively;
Figures 7a and 7b are schematic representations of the actuation mechanism shown in Figures 6a, 6b and 6c showing internal components and with jaws in closed positions;
Figures 8a and 8b are schematic representations of the actuation mechanism shown in Figures 6a, 6b and 6c showing internal components and with jaws in open positions;
Figures 9a and 9b are schematic representations of an actuation mechanism according to another embodiment of the first aspect of the invention showing front and back views respectively;
Figures 10a and 10b are schematic representations of an actuation mechanism according to another embodiment of the first aspect of the invention showing front and back views respectively; and
Figures 11a and 11b are schematic representations of an actuation mechanism according to another embodiment of the first aspect of the invention showing front and back views respectively.

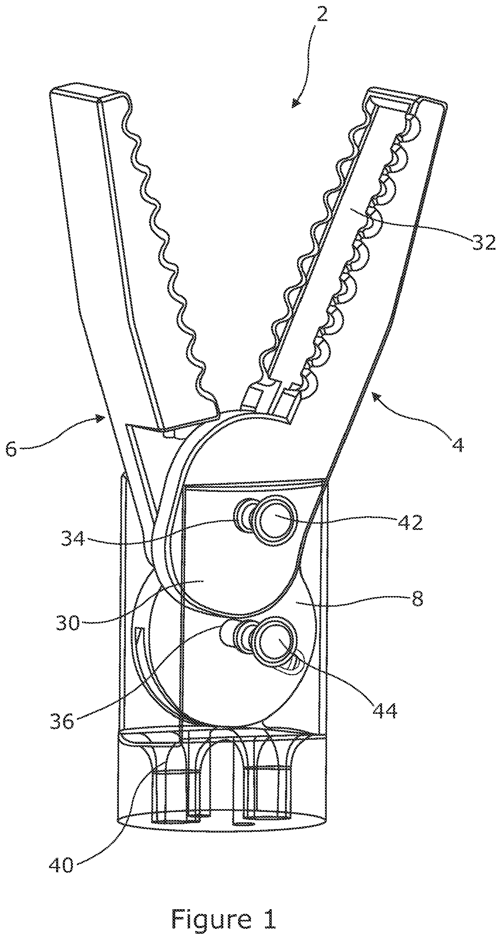

Referring initially to Figure 1, an actuation mechanism according to an embodiment of the invention is designated generally by the reference numeral 2. The actuation mechanism 2 comprises a first jaw 4, a second jaw 6, a driver 8 and a housing 40. Each jaw 4, 6 comprises a jaw axle receiving portion 34 and the housing 40 comprises a jaw axle 42 extending along a jaw axis and rotatably receivable within the jaw axle receiving portions 34 such that each jaw 4, 6 is rotatable relative to the housing 40 about the jaw axle 42.

Each jaw 4, 6 further comprises a tool portion 32 formed on the respective jaw. Each jaw is rotatable about the jaw axle 42 between an open position in which the tool portions are spaced apart and a closed position in which the tool portions contact one another. In this embodiment of the invention, the tool portions 32 of the first and second jaws 4, 6 are configured as graspers, but in other embodiments of the invention the tool portions may be configured to form part of any suitable tool such as scissors, forceps, or dissectors.

Each jaw 4, 6 also comprises a drive portion 30 engageable with the driver 8. The driver 8 comprises drive axle receiving portion 36 and the housing 40 further comprises a drive axle 44, extending along a drive axis parallel to the jaw axis. The drive axle 44 is rotatably receivable within the drive axle receiving portion 36 such that driver 8 is rotatable relative to the housing 40 about the drive axle 44.

Although the drive axis is spaced apart from the jaw axis, the overall drive mechanism comprising the driver and the drive portion of both jaws is compact. This contrasts with some known bipolar end effectors which comprise large linkage mechanisms to drive actuation of the jaws. The relative compactness of the driver 8 and drive portions 30 allows for more effective miniaturisation of the actuation mechanism 2 which may be particularly advantageous in the field of surgical robotics and devices for minimally invasive surgical procedures.

In this embodiment of the invention the driver 8 is disc shaped. However, in other embodiments of the invention the driver 8 may be any suitable shape provided it is rotatable about the drive axle 44.

Also, in this embodiment of the invention, the driver 8 is attachable to first and second tendons (not shown) which may each be actuated or pulled in order to rotate the driver 8 in different directions about the drive axis 44. In other embodiments of the invention the driver 8 may be attachable to any drive means suitable for rotating it about the drive axis. For example, the drive axis 44 may be fixed to the driver 8 and the drive axle 44 may be rotatable by a motor attached to the housing 40.

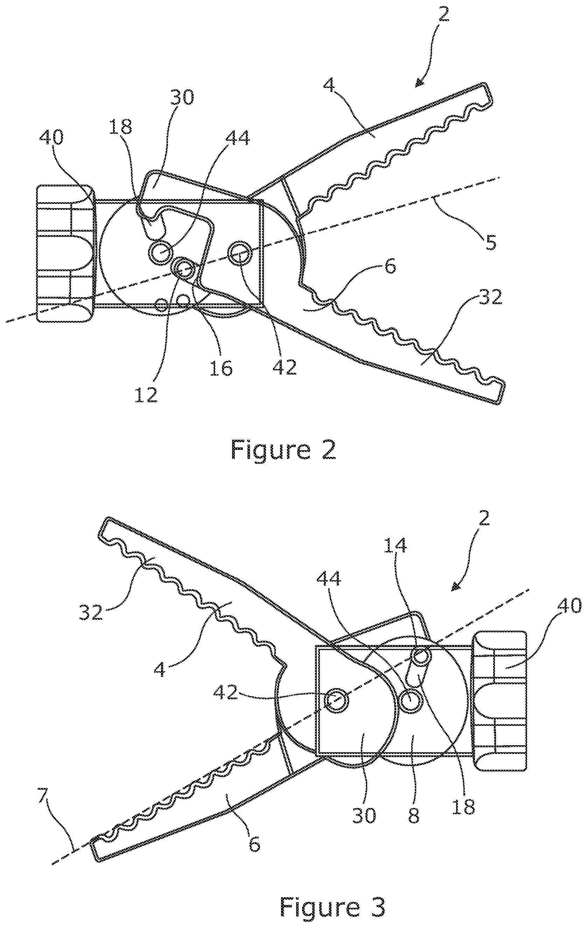

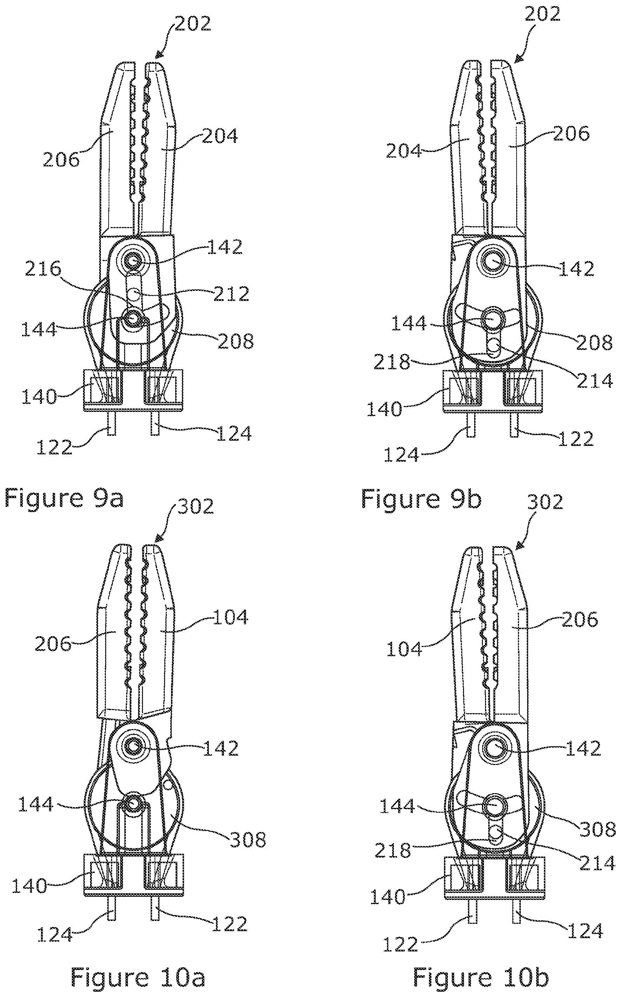

Figures 2 and 3 show each side of the actuation mechanism 2 of Figure 1. The first jaw 4 comprises a first jaw engagement portion, specifically a first pin 12, positioned in the first jaw's drive portion 30, the second jaw 6 comprises a second jaw engagement portion, specifically a second pin 14, positioned in the second jaw's drive portion 30 and the driver 8 comprises a first drive engagement portion (a first slot 16) and a second drive engagement portion (a second slot 18). In other embodiments of the invention, other arrangements of the pins and slots between the jaws and driver are possible. Examples of such other arrangements are described below with reference to Figures 9a to 11b.

The first pin 12 is slidably engageable with the first slot 16 and the second pin 14 is slidably engageable with the second slot 18. In use, when each pin 12, 14 is engaged with its respective slot 16, 18, rotation of the driver 8 about the drive axle 44 causes rotation of each jaw 4, 6 about the jaw axle 42 in an opposite direction to the other jaw. The rotation of the jaws 4, 6 occurs because the first and second pins 12, 14 are a fixed distance from the jaw axis, along which the jaw axle 42 extends, and the first and second slots 16, 18 are positioned and oriented in the driver such that as they rotate about the drive axis , along which the drive axle 44 extends, they cause the respective pins 4, 6 to rotate relative to the jaw axis while sliding within the slots.

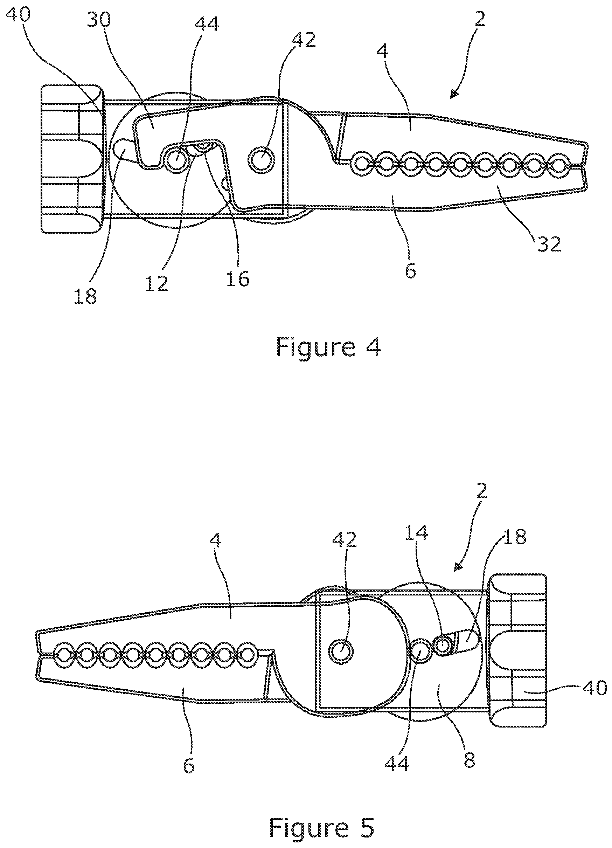

Further, each pin 12, 14 is positioned in the drive portion of the respective jaw and each slot 16, 18 is positioned and oriented in the driver such that when one of the jaws 4, 6 is in its open position the other jaw is also in its open position and when one of the jaws 4, 6 is in its closed position the other jaw ᵢs also in its closed position. This enables the actuation mechanism 2 to move between a fully open configuration and a fully closed configuration depending on the rotational position of the driver 8. Figures 2 and 3 demonstrate the open configuration and Figures 4 and 5 demonstrate the closed configuration of the jaws.

In this embodiment of the invention, the jaws 4, 6 move substantially symmetrically to one another. In other words, the range of movement of the first jaw 4 is substantially equal to that of the second jaw 106 so that, in the open configuration, the first jaw 4 and second jaw 6 have rotated substantially equal amounts from their respective closed configuration positions.

An advantage of the pin and slot mechanism is its simplicity and reliability. The entire actuation mechanism 2 consists of three moving parts only - the driver 8 and each jaw 4, 6. Also, the nature of the slidable engagement of each pin with its respective slot allows a margin of imprecision which may be off set merely by rotating the driver further in one sense or the other (i.e. clockwise or anticlockwise). Accordingly, the actuation mechanism 2 may be manufactured cost-effectively, especially in comparison to some known end effectors which require complex precision-engineered components in order to operate reliably and accurately.

Another, advantage of the pin and slot mechanism is that the force exerted by the driver 8 on each pin is variable based on the ratio between the radius of the driver 8 and the distance between each pin 12, 14 and the drive axis. This radius is proportional to a force amplification ratio for the respective jaw member 4, 6. In other words, the greater the ration between the radius of the driver and the distance between pin and axis, the greater the force that the respective jaw may exert when opening or closing. Increasing the force that the jaws may exert when closed may be particularly advantageous in surgical applications, for example maintaining a stong grip of a suturing needle.

A further advantage of the pin and slot mechanism is that the full range of movement achievable by the jaws may be caused by a small rotation of the driver (less than 180° for example) because each rotation of the driver causes rotation of both jaws in opposite directions so that the effect of the rotation is essentially doubled. This may allow for the movement of the jaws 4, 6 to be very responsive to actuations of the driver 8. Also, the size of the driver may vary depending on the application that the actuation mechanism 2 is to be used for. For example, the driver 8 may be made smaller in applications where compactness is preferable or made larger in applications where it is preferable for the jaws to generate greater rotational forces (i.e. where greater forces are required for grasping or cutting).

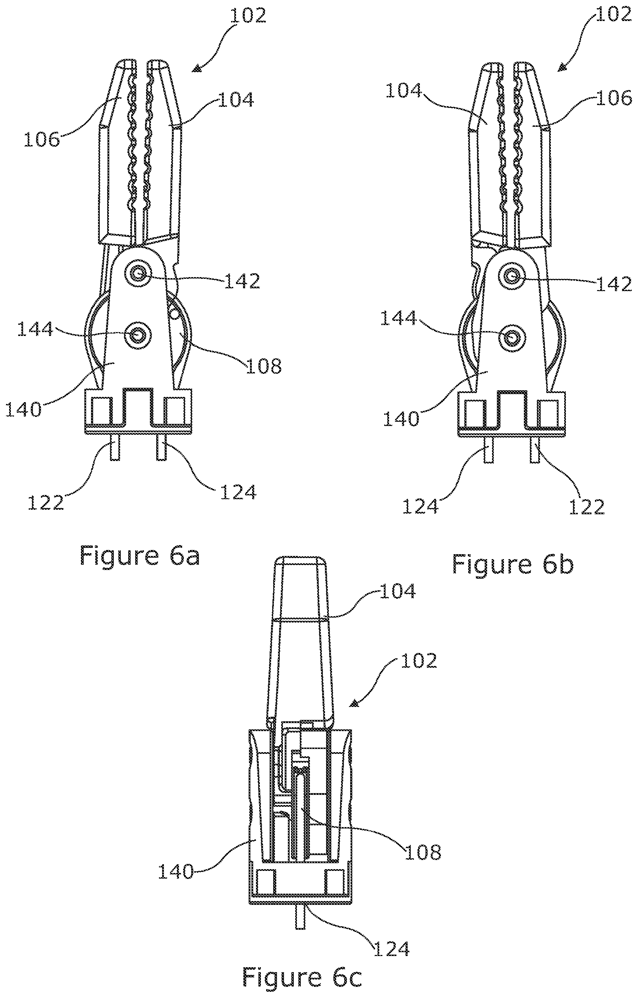

Referring now to Figures 6a, 6b and 6c, an actuation mechanism according to another embodiment of the invention is designated generally by the reference numeral 102. The actuation mechanism 102 is similar to the actuation mechanism 2 shown in Figures 1 to 5 and for ease of reference similar features have been given corresponding reference numerals. For example, the actuation mechanism 102 comprises a first jaw 104 similar to the first jaw 4 of the actuation mechanism 2 shown in Figure 1 **to** 5.

In this embodiment of the invention, the driver 108 is attachable to a first tendon 122 and a second tendon 124. Rotation of the driver 8 in a first **sense, to cause** rotation of the jaws 104, 106 towards closed positions for example, may be achieved by actuating or pulling the first tendon 122. Conversely, rotation of the driver 8 in a second sense, to cause rotation of the jaws 104, 106 towards open positions for example, may be achieved by actuating or pulling the second tendon 122.

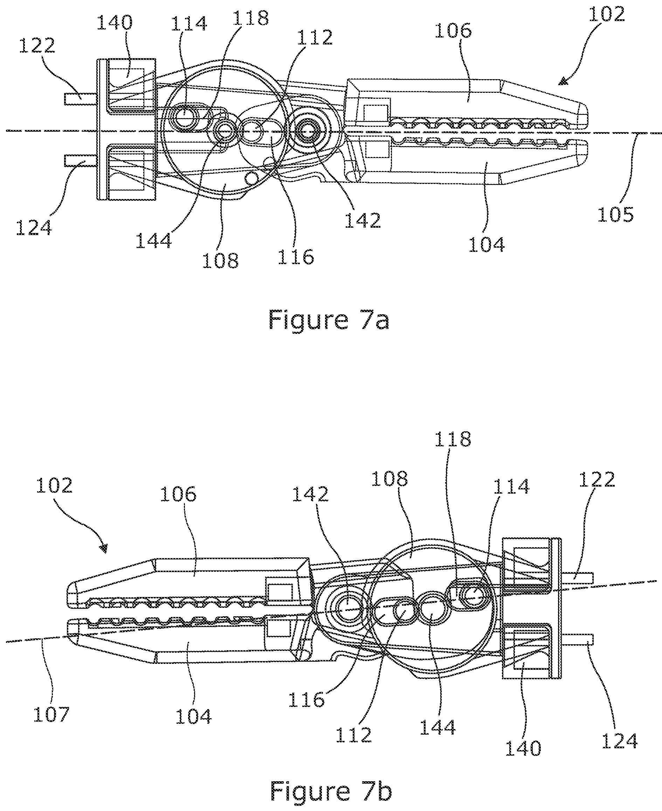

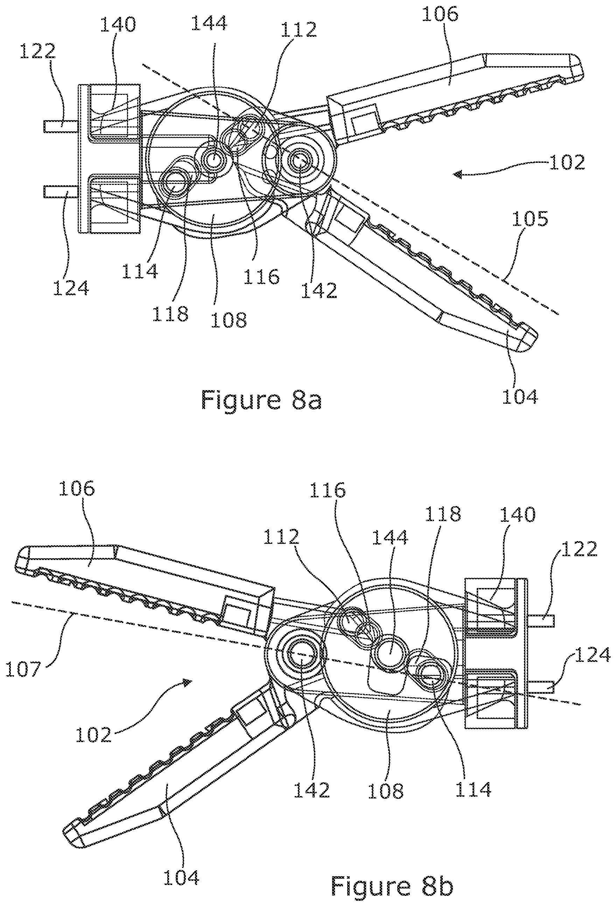

Accordingly, a single pair of antagonistic tendons (first and second tendons 122, 124) may be used to actuate the actuation mechanism 102 between an closed configuration, shown in Figures 7a and 7b, and a closed configuration, shown in Figures 8a and 8b.

In Figures 7a and 7b, the first jaw 104 comprises a first pin 112, the second jaw comprises a second pin 114 and the driver 108 comprises first and second slots 116, 118 (similarly to the actuation mechanism 2 shown in Figures 1 to 5). Highlighted in Figure 7a is a first jaw centre line 105 that extends through the jaw axis 142, along which the jaw axle 142 extends, and the centre of the first pin 112. Meanwhile, highlighted in Figure 7b is a second jaw centre line 107 that extends through the jaw axis and the centre of the second pin 114.

In Figures 8a and 8b, the first tendon 122 has been pulled to cause the driver 108 to rotate about the drive axis relative to the position it held in Figures 7a and 7b and cause the jaws 104, 106 to rotate such that the actuation mechanism 102 is now in the open configuration. In Figure 8a the driver 108 has rotated anti-clockwise relative to the orientation it had in Figure 7a while the first jaw 104 and first jaw centre line 105 have rotated clockwise about the jaw axle 142. In Figure 8b the driver 108 has rotated clockwise relative to the orientation it had in Figure 7b and the second jaw 106 and second jaw centre line 107 have also rotated clockwise about the jaw axle 142.

In this embodiment of the invention, the jaws 104, 106 move asymmetrically to one another. In other words, the range of movement of the first jaw 104 is greater than that of the second jaw 106 so that, in the open configuration, the first jaw 104 has rotated further from its closed configuration position than the second jaw 106 has.

In use, a force may be exerted by one of the tendons 122, 124 on the driver 108 to cause it to rotate, refered to herein as a tendon force. The driver 108 transmits the tendon force to each jaw 104, 106 via each slots 116, 118 pushing against its respective pin 112, 114. The force exerted on the pins 112, 114 by the slots 116, 118 is refered to herein as a driving force and acts normally to the orientation of the slot. As each jaw 104, 106 pivots about the jaw axle 142, the driving force is transmitted to a force exerted by each tool portion. This force is the force with which the jaws open and close and is referred to herein as the gripping force. The gripping force exerted by each jaw acts normally to the respective jaw centreline 105, 107. Therefore, the proportion of each driving force that acts normally to the respective jaw centre line 105, 107 will be transmitted to the gripping force, but any proportion of the driving that acts parallel to the respective jaw cetre line will be lost as friction.

Accordingly, an advantage of the pin and slot mechanism is that the gripping force can be inceased where strong force is required to be exerted by the tool portions.

Referring now to Figures 9a and 9b, an actuation mechanism according to another embodiment of the invention is designated generally by the reference numeral 202. Some parts of the actuation mechanism 202 correspond to parts of the actuation mechanism 102 shown in Figures 6a to 8b and have therefore been given the same reference numerals for ease of reference.

The actuation mechanism 202 differs from the actuation mechanism 102 shown in Figures 6a to 8b in that first and second drive engagement portions comprise first and second pins 212, 214 and the first and second jaw engagement portions comprise first and second slots 216, 218, rather than vice versa as is the case for the actuation mechanism 2 shown in Figures 1 to 5. In other words, the first jaw 204 comprises the first slot 216, the second jaw comprises the second slot 218 and the driver 208 comprises the first and second pins 212, 214. The first pin 212 is slidably engageable with the first slot 216 and the second pin 114 is slidably engageable with the second slot 218 such that rotation of the first and second jaws 204, 206 is achievable by rotating the driver 208. In this embodiment of the invention rotation of the first and second pins 212, 214 about the drive axis, along which the drive axle 144 extends, causes the respective slots 216, 218 to rotate relative to the jaw axis, along which the jaw axle 142 extends, in order that each pin 212, 214 remains within its slot 216, 218. The driver 208 is attachable to a first tendon 122 and a second tendon 124, similarly to the driver 108 shown in figures 6a to 8b.

In other embodiments of the invention it is possible to vary the pin and slot mechanism so that the driver comprises one pin and one slot, rather than both pins or both slots as described above.

For example, in Figures 10a and 10b, an actuation mechanism according to an embodiment of the invention is designated generally by the reference numeral 302. The actuation mechanism 302 comprises a first jaw 104 and a second jaw 206. The actuation mechanism 302 comprises a driver 308 adapted to engage with the first and second jaws 104, 206. The driver 308 therefore comprises a first slot (not shown, but similar to the first slot 116 shown in Figures 7a to 8b) engageable with the first pin (not shown here, but shown in Figures 7a to 8b) forming part of the first jaw 104. The driver 308 also comprises a second pin 214, identical to the second pin 214 shown in Figures 91 and 9b, engageable with the second slot 218 of the second jaw 206.

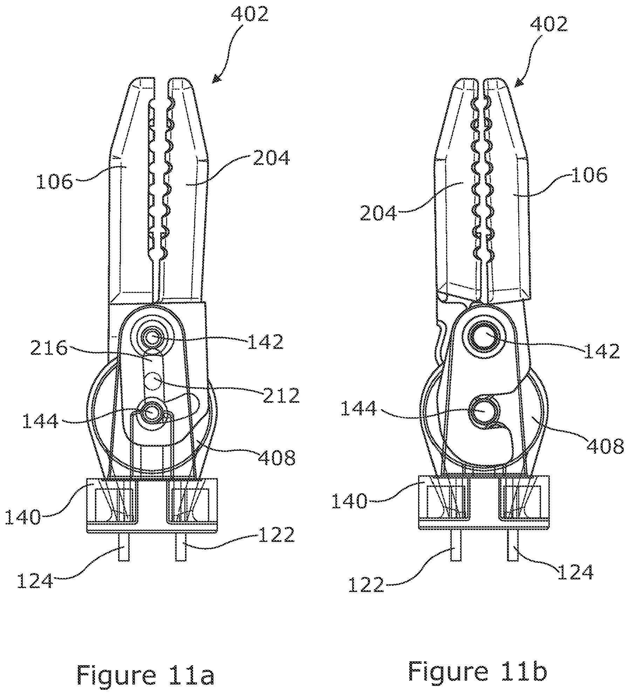

Alternatively, in Figures 11a and 11b, an actuation mechanism according to an embodiment of the invention is designated generally by the reference numeral 402. The actuation mechanism 402 comprises a first jaw 204 and a second jaw 106. The actuation mechanism 402 comprises a driver 408 adapted to engage with the first and second jaws 204, 106. The driver 308 therefore comprises a first pin 212 engageable with the first slot 216 forming part of the first jaw 204. The driver 308 also comprises a second slot (not shown, but similar to the second slot 118 shown in Figures 7a to 8b) engageable with the second pin 118 (not shown here, but shown in Figures 7a to 8b) of the second jaw 106.

The scope of the invention is defined by the invention.

## Claims

1. An actuation mechanism (2) comprising:
a first jaw (4) rotatable about a jaw axis and comprising a first jaw engagement portion,
a second jaw (6) rotatable about the jaw axis and comprising a second jaw engagement portion, and
a driver (8) rotatable about a drive axis parallel to the jaw axis and comprising a first drive engagement portion and a second drive engagement portion; wherein the first jaw engagement portion and the first drive engagement portion are slidably engageable with one another and the second jaw engagement portion and the second drive engagement portion are slidably engageable with one another; whereby, when the first jaw engagement portion is engaged with the first drive engagement portion and the second jaw engagement portion is engaged with the second drive engagement portion, rotation of the driver (8) about the drive axis causes rotation of the first jaw (4) in a first sense and the second jaw (6) in an opposite sense,
wherein one of the first jaw engagement portion and the first drive engagement portion comprises a first pin (12), and the other one comprises a first radially extending slot (16); and one of the second jaw engagement portion and the second drive engagement portion comprises a second pin (14), and the other one comprises a second radially extending slot (18).

2. An actuation mechanism (2) according to claim 1, further comprising a first (122) and second tendon (124) each attachable to the driver.

3. An actuation mechanism (2) according to claim 1 or 2, wherein each jaw (4 ,6) comprises a drive portion (30), a tool portion (32) and a jaw axle receiving portion (36) positioned between the drive portion (30) and the tool portion (32) and extending along the jaw axis.

4. An actuation mechanism (2) according to claim 3, wherein the first and second jaw engagement portions are each positioned in the drive portion (30) of the respective jaw (4, 6).

5. An actuation mechanism (2) according to claim 3 or claim 4, wherein the tool portions (32) of the first (4) and second jaws (6) are configured as scissor blades, forceps, a dissector or a grasper.

6. An actuation mechanism (2) according to any of claim 3 to claim 5, wherein each jaw (4, 6) is rotatable between an open position and a closed position.

7. An actuation mechanism (2) according to claim 6, wherein the actuation mechanism (2) is movable between an open configuration, in which each jaw (4, 6) is in its open position, and a closed configuration, in which each jaw is in its closed position.

8. An actuation mechanism (2) according to any of claim 3 to claim 7, further comprising a housing (40) comprising a jaw axle (42) extending along the jaw axis and rotatably receivable within the jaw axle receiving portion (34) of each jaw (4, 6) such that each jaw (4 , 6) is rotatable relative to the housing (40) about the jaw axle (42).

9. An actuation mechanism (2) according to 8, wherein the driver (8) further comprises a drive axle receiving portion (36) and the housing (40) further comprises a drive axle (44) extending along the drive axis and rotatably receivable within the drive axle receiving portion (36) of the driver (8) such that the driver (8) is rotatable relative to the housing (40) about the drive axle (44).

10. A surgical instrument comprising a shaft, an articulated portion coupled to the shaft and an end effector coupled to the articulated portion, which end effector comprises an actuation mechanism according to any of the preceding claims.

## Patentansprüche

1. Betätigungsmechanismus (2) umfassend:
eine erste Backe (4), die um eine Backenachse drehbar ist und einen ersten Backeneingriffsteil umfasst,
eine zweite Backe (6), die um die Backenachse drehbar ist und einen zweiten Backeneingriffsteil umfasst, und
einen Treiber (8), der um eine Antriebsachse parallel zur Backenachse drehbar ist und ein erstes Antriebseingriffsteil und ein zweites Antriebseingriffsteil umfasst; wobei der erste Backeneingriffsteil und das erste Antriebseingriffsteil gleitend miteinander in Eingriff bringbar sind und der zweite Backeneingriffsteil und das zweite Antriebseingriffsteil gleitend miteinander in Eingriff bringbar sind; wodurch, wenn der erste Backeneingriffsteil mit dem ersten Antriebseingriffsteil in Eingriff ist und der zweite Backeneingriffsteil mit dem zweiten Antriebseingriffsteil in Eingriff ist, die Drehung des Treibers (8) um die Antriebsachse eine Drehung der ersten Backe (4) in eine erste Richtung und der zweiten Backe (6) in eine entgegengesetzte Richtung bewirkt,
wobei einer von dem ersten Backeneingriffsteil und dem ersten Antriebseingriffsteil einen ersten Stift (12) umfasst, und der andere einen ersten sich radial erstreckenden Schlitz (16) umfasst; und
einer von dem zweiten Backeneingriffsteil und dem zweiten Antriebseingriffsteil einen zweiten Stift (14) umfasst, und der andere einen zweiten sich radial erstreckenden Schlitz (18) umfasst.

2. Betätigungsmechanismus (2) nach Anspruch 1, ferner umfassend eine erste (122) und zweite Sehne (124), die jeweils an dem Treiber befestigbar sind.

3. Betätigungsmechanismus (2) nach Anspruch 1 oder 2, wobei jede Backe (4, 6) einen Antriebsteil (30), einen Werkzeugteil (32) und einen Backenachsenaufnahmeabschnitt (36) umfasst, der zwischen dem Antriebsteil (30) und dem Werkzeugteil (32) positioniert ist und sich entlang der Backenachse erstreckt.

4. Betätigungsmechanismus (2) nach Anspruch 3, wobei der erste und zweite Backeneingriffsteil jeweils in dem Antriebsteil (30) der jeweiligen Backe (4, 6) positioniert sind.

5. Betätigungsmechanismus (2) nach Anspruch 3 oder Anspruch 4, wobei die Werkzeugteile (32) der ersten (4) und zweiten Backen (6) als Scherenklingen, Zange, ein Dissektor oder ein Greifer konfiguriert sind.

6. Betätigungsmechanismus (2) nach einem der Ansprüche 3 bis 5, wobei jede Backe (4, 6) zwischen einer offenen Position und einer geschlossenen Position drehbar ist.

7. Betätigungsmechanismus (2) nach Anspruch 6, wobei der Betätigungsmechanismus (2) zwischen einer offenen Konfiguration, in der jede Backe (4, 6) in ihrer offenen Position ist, und einer geschlossenen Konfiguration, in der jede Backe in ihrer geschlossenen Position ist, bewegbar ist.

8. Betätigungsmechanismus (2) nach einem der Ansprüche 3 bis 7, ferner umfassend ein Gehäuse (40), das eine Backenachse (42) umfasst, die sich entlang der Backenachse erstreckt und drehbar in dem Backenachsenaufnahmeabschnitt (34) jeder Backe (4, 6) aufnehmbar ist, sodass jede Backe (4, 6) relativ zu dem Gehäuse (40) um die Backenachse (42) drehbar ist.

9. Betätigungsmechanismus (2) nach Anspruch 8, wobei der Treiber (8) ferner einen Triebachsenaufnahmeabschnitt (36) umfasst und das Gehäuse (40) ferner eine Triebachse (44) umfasst, die sich entlang der Antriebsachse erstreckt und drehbar in dem Triebachsenaufnahmeabschnitt (36) des Treibers (8) aufnehmbar ist, sodass der Treiber (8) relativ zu dem Gehäuse (40) um die Triebachse (44) drehbar ist.

10. Chirurgisches Instrument umfassend einen Schaft, einen gelenkigen Abschnitt, der mit dem Schaft gekoppelt ist, und einen Endeffektor, der mit dem gelenkigen Abschnitt gekoppelt ist, wobei der Endeffektor einen Betätigungsmechanismus nach einem der vorhergehenden Ansprüche umfasst.

## Revendications

1. Mécanisme d'actionnement (2) comprenant :
une première mâchoire (4) rotative autour d'un axe de mâchoire et comprenant une première partie d'engagement de mâchoire,
une deuxième mâchoire (6) rotative autour de l'axe de mâchoire et comprenant une deuxième partie d'engagement de mâchoire, et
un entraîneur (8) rotatif autour d'un axe d'entraînement parallèle à l'axe de mâchoire et comprenant une première partie d'engagement d'entraînement et une deuxième partie d'engagement d'entraînement ; dans lequel la première partie d'engagement de mâchoire et la première partie d'engagement d'entraînement sont engageables de façon coulissante l'une avec l'autre et la deuxième partie d'engagement de mâchoire et la deuxième partie d'engagement d'entraînement sont engageables de façon coulissante l'une avec l'autre ; de sorte que, lorsque la première partie d'engagement de mâchoire est engagée avec la première partie d'engagement d'entraînement et la deuxième partie d'engagement de mâchoire est engagée avec la deuxième partie d'engagement d'entraînement, la rotation de l'entraîneur (8) autour de l'axe d'entraînement provoque la rotation de la première mâchoire (4) dans un premier sens et de la deuxième mâchoire (6) dans un sens opposé,
dans lequel l'une de la première partie d'engagement de mâchoire et de la première partie d'engagement d'entraînement comprend une première broche (12), et l'autre comprend une première fente s'étendant radialement (16) ; et l'une de la deuxième partie d'engagement de mâchoire et de la deuxième partie d'engagement d'entraînement comprend une deuxième broche (14), et l'autre comprend une deuxième fente s'étendant radialement (18).

2. Mécanisme d'actionnement (2) selon la revendication 1, comprenant en outre un premier (122) et un deuxième tendon (124) chacun pouvant être fixé à l'entraîneur.

3. Mécanisme d'actionnement (2) selon la revendication 1 ou 2, dans lequel chaque mâchoire (4, 6) comprend une partie d'entraînement (30), une partie d'outil (32) et une partie de réception d'axe de mâchoire (36) qui est positionnée entre la partie d'entraînement (30) et la partie d'outil (32) et qui s'étend le long de l'axe de mâchoire.

4. Mécanisme d'actionnement (2) selon la revendication 3, dans lequel les première et deuxième parties d'engagement de mâchoire sont chacune positionnées dans la partie d'entraînement (30) de la mâchoire respective (4, 6).

5. Mécanisme d'actionnement (2) selon la revendication 3 ou la revendication 4, dans lequel les parties d'outil (32) des première (4) et deuxième mâchoires (6) sont configurées comme des lames de ciseaux, un forceps, un dissecteur ou un préhenseur.

6. Mécanisme d'actionnement (2) selon l'une quelconque des revendications 3 à 5, dans lequel chaque mâchoire (4, 6) est rotative entre une position ouverte et une position fermée.

7. Mécanisme d'actionnement (2) selon la revendication 6, dans lequel le mécanisme d'actionnement (2) est mobile entre une configuration ouverte, dans laquelle chaque mâchoire (4, 6) est dans sa position ouverte, et une configuration fermée, dans laquelle chaque mâchoire est dans sa position fermée.

8. Mécanisme d'actionnement (2) selon l'une quelconque des revendications 3 à 7, comprenant en outre un boîtier (40) comprenant un axe de mâchoire (42) qui s'étend le long de l'axe de mâchoire et qui est recevable de façon rotative dans la partie de réception d'axe de mâchoire (34) de chaque mâchoire (4, 6) de sorte que chaque mâchoire (4, 6) est rotative par rapport au boîtier (40) autour de l'axe de mâchoire (42).

9. Mécanisme d'actionnement (2) selon la revendication 8, dans lequel l'entraîneur (8) comprend en outre une partie de réception d'essieu moteur (36) et le boîtier (40) comprend en outre un essieu moteur (44) qui s'étend le long de l'axe d'entraînement et qui est recevable de façon rotative dans la partie de réception d'essieu moteur (36) de l'entraîneur (8) de sorte que l'entraîneur (8) est rotatif par rapport au boîtier (40) autour de l'essieu moteur (44).

10. Instrument chirurgical comprenant une tige, une partie articulée couplée à la tige et un effecteur terminal couplé à la partie articulée, lequel effecteur terminal comprend un mécanisme d'actionnement selon l'une quelconque des revendications précédentes.
